# EUROPEAN PATENT SPECIFICATION

(11) **EP 1 124 789 B1**
(45) Date of publication and mention of the grant of the patent: **29.09.2004**
(21) Application number: 98956379.6
(22) Date of filing: 30.10.1998
(51) Int. Cl.: C07C 231/06

(54) **PRODUCTION OF AMIDES AND/OR ACIDS FROM NITRILES**
VERFAHREN ZUR HERSTELLUNG VON AMIDEN UND/ODER SÄUREN VON NITRILEN
PRODUCTION D'AMIDES ET/OU D'ACIDES A PARTIR DE NITRILES

(43) Date of publication of application: 22.08.2001
(73) Proprietor: Catalytic Distillation Technologies, Pasadena, Texas 77507 (US)
(72) Inventor: BEZUIDENHOUT, Barend, Christiaan, Buurman, Sasolburg (ZA); DENGA, Zamile, Edendale Johannesburg (ZA); STEYN, Rian, Bredel Kempton Park (ZA); STEYNBERG, Petrus, Johannes, Sasolburg (ZA); STARK, Nicolaus, Ladislaus, Vanderbijlpark (ZA)
(74) Representative: Ebner von Eschenbach, Jennifer
(86) International application number: PCT/US1998/023083
(87) International publication number: WO 2000/026178

(56) References cited:
- EP-A- 0 461 850
- EP-A- 0 635 484
- EP-A- 0 670 301
- GB-A- 831 051
- GB-A- 1 315 530
- US-A- 3 923 741
- US-A- 3 941 837
- US-A- 5 276 185

## Description

This invention relates to the production of amides and/or acids from nitriles. It relates in particular to a process for producing an amide and/or acid from a nitrile.

Documents EP 0 670 301, EP 0 635 484, GB 1 315 530, EP 0 461 850, US 5 276 185, GB 831 051, US 3 941 837 and US 3 923 741 disclose the hydration of a nitrile in the presence of a catalyst. Distillation can be carried out as a means of purification.

### SUMMARY OF THE INVENTION

According to the invention, there is provided a process for producing an amide and/or acid from a nitrile, which process comprises introducing a nitrile, as a first reactant, and a hydration compound, as a second reactant which is capable of reacting with the nitrile to convert it to its corresponding amide thus hydrating the nitrile and/or to convert it to its corresponding acid, into a treatment zone in a column or tower, with the catalyst bed provided in a section of the tower, and with a distillation zone being provided above and below the catalyst bed; and comprising at least one reaction zone in which the hydration reaction of the nitrile to the amide and/or acid takes places catalytically in the presence of a catalyst, and at least one distillation zone adjacent the reaction zone in which distillation of the reaction product(s) from the reaction zone and/or unreacted reactants, takes place, with the reaction zone comprising a packed bed of particles of a copper or copper-based hydration catalyst; effecting hydration of at least some of the nitrile to the corresponding amide, and/or its corresponding acid; said hydration being effected simultaneously with distillation in a single treatment zone containing both liquid and vapor throughout; withdrawing the amide and/or acid from the treatment zone in a concentration of 1-60%.

Catalytic distillation thus involves effecting a chemical reaction simultaneously with or in combination with distillation, in a single treatment zone. The treatment zone will thus comprise at least one reaction zone in which the hydration reaction of the nitrile to the amide and/or acid takes place catalytically in the presence of a catalyst, and at least one distillation zone adjacent the reaction zone in which distillation of the reaction product(s) from the reaction zone and/or unreacted reactants, takes place.

The reaction zone may thus comprise a packed bed of catalyst particles capable of catalyzing the conversion or hydration of the nitrile to its corresponding amide. Any suitable hydration catalyst can be used, typically a copper or copper-based hydration catalyst, e.g. a copper-chromium or a copper oxide hydration catalyst.

The first reactant may comprise an unsaturated or aromatic nitrile, such as acrylonitrile, methacrylonitrile, crotononitrile, allyl cyanide, or benzonitrile, which will thus be hydrated to the corresponding unsaturated or aromatic amide and/or acid, without a substantial degree of polymerization taking place. Instead, however, the first reactant may comprise a saturated nitrile such as acetonitrile, propionitrile, butyronitrile, or isobutyronitrile.

The treatment zone will typically be provided in a column or tower, with the catalyst bed provided in a section of the tower. The distillation zone may thus be provided above and/or below the catalyst bed. Preferably, a distillation zone is provided above and below the catalyst bed. Suitable packed distillation media, e.g. Raschig rings, or distillation apparatus or equipment, are then provided in the column below and/or above the catalyst bed, i.e. in the distillation zone(s).

The process may include boiling a liquid component in a reboiling zone operatively connected to a lower end of the treatment zone, to provide the driving force for the catalytic distillation. A portion of the liquid component may then, if desired, be introduced into the treatment zone, e.g. above or below the catalyst bed.

The liquid component may be such that it does not partake in the hydration reaction i.e. it only provides the driving force for the catalytic distillation and thus assists in distillation of the reactants and products in the treatment zone. In such case, the second reactant may be fed into the treatment zone at a location spaced from the point of introduction of the first reactant or nitrile into the treatment zone, e.g. above the catalyst bed when the nitrile is fed into the treatment zone below the catalyst bed. The second reactant must thus be capable of hydrating the nitrile at the conditions prevailing in the treatment zone and in the presence of the catalyst. In particular, the second reactant may be water.

The liquid component may be an organic compound such as an alcohol, an aromatic or a paraffin.

However, the liquid component may, instead, be such that it partakes in the hydration reaction. It may thus, in particular, be the same as the second reactant. In other words, some second reactant is then used for reboiling, while some thereof is introduced into the treatment zone as hereinbefore described.

The higher boiling of the first and second reactants may be introduced into the treatment zone above the catalyst bed, with the lower boiling being introduced below or above the catalyst bed. In the event that the first reactant or nitrile is the higher boiling component, a portion thereof will thus be introduced above the catalyst bed, while remainder thereof will be boiled in the boiling zone to provide the driving force for the catalytic distillation.

The withdrawal of the amide may be effected as an overhead or distillate component at the top of the treatment zone or as a high boiling component at the bottom of the treatment zone, e.g. from the reboiling zone, depending on the relative boiling points of the first and second reactants.

The column may be of any desired length and width, and is typically in the region of 10m to 60m long. Typically, its diameter is in the region of 25mm to 110mm on a pilot plant scale and greater than 110m for a commercial scale operation. The catalyst bed may also be of any desired length, e.g. 0.5-10m. The pressure in the column can vary widely, e.g. between 10kPa(g) and 10000kPa(g), and can be controlled by means of an inert gas such as nitrogen or argon. The pressure, and hence the reaction temperature, in the column will determine the product produced. Thus, if an amide corresponding to the nitrile which is fed into the column, is produced at a given column pressure and hence a specific reaction temperature, the corresponding acid can instead, or additionally, be produced by increasing the column pressure and hence the reaction temperature at which the reaction is effected, so that over or excessive hydrolysis is effected, thereby forming the corresponding acid.

### BRIEF DESCRIPTION OF THE INVENTION

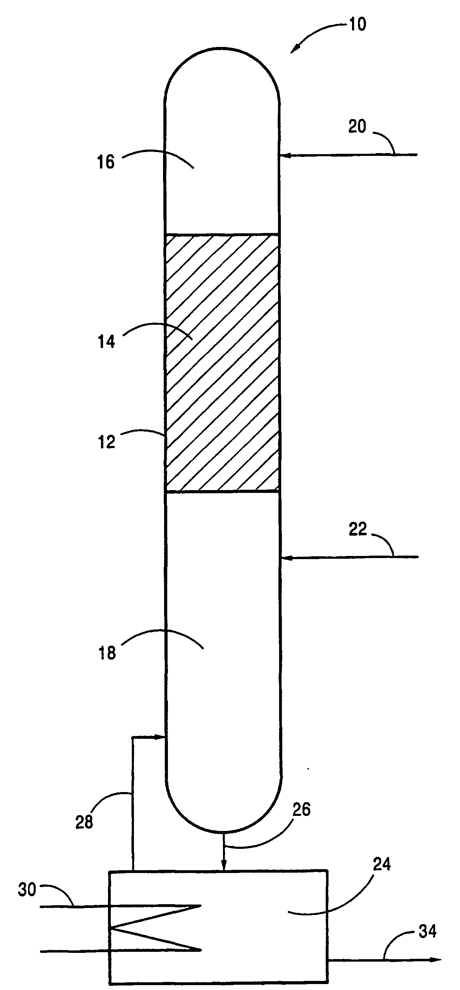

The figure is a diagrammatic drawing which shows a simplified flow diagram of a process according to the invention.

### DETAILED DESCRIPTION

The invention will now be described in more detail, with reference to the accompanying diagrammatic drawing which shows a simplified flow diagram of a process according to the invention for producing an amide and/or an acid from a nitrile, as well as the subsequent Examples.

In the drawing, reference numeral 10 generally indicates a process according to the invention for producing an amide from a nitrile.

The process 10 includes a catalytic distillation column 12. The dimensions of the column 12 can vary widely, but it is typically 10m long with an internal diameter of 25mm.

A reaction zone 14 is provided inside the column 12 such that a distillation zone 16 is provided above the zone 14 while another distillation zone 18 is provided below the reaction zone 14. The reaction zone 14 comprises a supported bed of a copper based particulate hydration catalyst such as a supported copper-chromium catalyst, a supported copper oxide catalyst or another similar hydration catalyst. The distillation zones 16, 18 are packed with Raschig rings (not shown).

A water feedline 20 leads into the column 12 above the bed 14, while a nitrile feedline 22 leads into the column 12 immediately below the bed 14. However, it is to be appreciated that the nitrile feedline 22 can also lead into the column 12 above the bed 14.

A reboiler 24 is located below the column 12. A withdrawal line 26 leads from the bottom of the column 12 to the reboiler 24, while a return line 28 leads from the reboiler 24 back to the column 12. The reboiler 24 is fitted with a heater 30, while a product withdrawal line 34 leads from the reboiler.

In use, sufficient water is introduced into the reboiler 24 to fill it to 30-80% of its volumetric capacity, and heated. The pressure in the column 12 is regulated at between 0.1 and 100 bar, as desired, by means of an inert gas such as nitrogen or argon. The water in the reboiler 24 is boiled up into the column 12 until full reflux is reached. At that stage, a feedstream of a nitrile, such as acrylonitrile which has a lower boiling point than water, is introduced into the column 22 along the feedline 22, typically at the rate of between 0.001 and 50kg per hour, followed by the introduction of water along the flowline 20 at a suitable feed rate, e.g. between 0.001 and 100kg per hour. Typically, the nitrile used as feedstock is stabilized against polymerization with radical inhibitors such as hydroquinone or methylated hydroquinone, before introduction thereof into the column. The column 12 is kept under conditions of reflux, and an amide product of the nitrile, together with excess water, is recovered as the bottom stream along the flowline 34, at a rate of 0.002kg to 150kg per hour.

In simulations of the process 10, the following nonlimiting examples was conducted in the laboratory.

### EXAMPLE 1

Pellets of a copper-chromite catalyst in its reduced form (650g), supported in stainless steel wire socks (22 in number), were packed in a 5 m section of a catalytic distillation column 12 having dimensions of 10m height x 25mm diameter. The top one meter of the column (zone 16) and the bottom 4 meters (zone 18) were filled with Raschig rings. Demineralized water was introduced into the reboiler 24 to 30% of its capacity. Under a nitrogen atmosphere, the water was boiled up into the column under atmospheric pressure (85kPa) until a reflux was reached (96°C). Acrylonitrile containing 35ppm methylated hydroquinone (MeHQ) was introduced at a feed point(flow line 22) just below the catalyst bed at a rate of 30g/hour and water fed (flow line 20) above the catalyst zone at a rate of 84g/hour. After the introduction of acrylonitrile, the temperature inside the catalyst bed dropped to the boiling point of the acrylonitrile-water azeotrope (64°C). The product solution containing 35% by weight acrylamide (100% conversion and 100% selectivity) was removed from the reboiler along the flow line 34, at a rate of 114g/hour.

### EXAMPLE 2

Extrudates or pellets of a copper oxide or copper-chromite catalyst in reduced form (350g), supported in stainless steel wire socks (10 in number) and wrapped in demister wire, were packed in the top section of a glass catalytic distillation column having dimensions of 2.1m height x 35mm diameter. The bottom 600mm of the column was filled with Raschig rings or structured distillation packing. De-aerated demineralized water was introduced into the reboiler to 30% of its capacity. Under a nitrogen atmosphere, the water was boiled up into the column under atmospheric pressure (85kPa), until reflux was reached (96°C). De-aerated nitrile was introduced at a feed point just below the catalyst bed at a rate of 10-25g/hour, and water was fed into the column above the catalyst zone at the rate required to produce the desired product concentration. After the introduction of the nitrile, the temperature inside the catalyst bed decreased to the boiling point of the nitrile-water azeotrope. The product solution (25-130g/h) containing up to 50% by weight of the amide (>90% conversion and selectivity) was removed from the reboiler.

### EXAMPLE 3

Extrudates of a copper oxide catalyst in its reduced form (900g), supported in stainless steel wire socks (22 in number) and wrapped in demister wire, were packed in a 8.5m section of a catalytic distillation column having dimensions of 10m height x 25mm diameter. The bottom 1.5m of the column was filled with 10mm Berl saddles. De-aerated demineralized water was introduced into the reboiler to 30% of its capacity. Under a nitrogen atmosphere of 200kPa above atmospheric pressure, the water was boiled up into the column until reflux was reached (135°C). Deaerated acrylonitrile (containing 35ppm MeHQ) was introduced at a feed point just below the catalyst bed at a rate of 48-152g/hour, and water was fed into the column above the catalyst zone at such a rate as to produce the required product concentration. After the introduction of acrylonitrile, the temperature inside the catalyst bed decreased to the boiling point of the acrylonitrile-water azeotrope (104°C). The product solution containing up to 50% by weight acrylamide (>98% conversion and selectivity) was removed from the reboiler at a rate of 200-500g/hour.

### EXAMPLE 4

In this example, the column set-up and catalyst packing were the same as for Example 3, but the nitrogen pressure inside the column was raised to 400kPa above atmospheric pressure, which resulted in the reboiler temperature being 158°C. When the acrylonitrile (180g/h) was introduced above the catalyst zone, the temperature in the catalyst zone decreased to 135°C-145°C and an aqueous solution of acrylic acid (75g/h) and acrylamide (175g/h) was produced.

### EXAMPLE 5

Extrudates of a copper oxide catalyst in its reduced form (13.5kg), supported in stainless steel wire socks and wrapped in demister wire, were packed in a 7m section of a catalytic distillation column having dimensions of 10m height x 110mm diameter. The bottom 2m of the column was filled with 10mm Berl saddles. De-aerated demineralized water was introduced into the reboiler to 50% capacity. Under a nitrogen atmosphere of 100kPa above atmospheric pressure, the water was boiled up into the column until reflux was reached (121°C). Deaerated acrylonitrile containing 35ppm MeHQ was introduced at a feed point above the catalyst bed at a rate of 0.5-2.5g/hour, and water was fed into the column above the catalyst zone at such a rate as to produce the required product concentration. After the introduction of acrylonitrile, the temperature inside the catalyst bed decreased to a boiling point of the acrylonitrile-water azeotrope (89°C). The pH of the product solution was controlled between 5.0 and 6.0 by the addition of a 0.0125M sulfuric acid solution into the reboiler. The product solution containing up to 50% by weight acrylamide (>98% conversion and selectivity) was removed from the reboiler at a rate of 5-30kg/hour.

It is known to produce amides from nitriles by hydration of the nitriles in batch, fixed or slurry bed reactors. Three types of reactions are known, namely:
a) Homogeneous, mainly sulfuric acid, catalyzed reactions;
b) Heterogeneous catalyzed reactions with copper or copper based metal oxide mixtures, e.g. copper oxide or chromium oxide, as catalysts;
c) Reactions in which biocatalysts such as enzymes are used to facilitate the hydration of the nitriles.

These reactions are used for the production of amides, such as for the production of an acrylamide monomer from a nitrile such as acrylonitrile. Such monomers in turn are used for the production of water soluble polymers and copolymers which are used as mining flocculants, paper making aids, thickening agents, surface coatings and enhanced oil recovery products.

The Applicant is aware that in the mainly sulfuric acid catalyzed batch processes, the highly exothermic hydration reaction of nitriles is complicated by polymer formation, if the reaction temperature and reactant ratios are not controlled carefully. To end the reaction, the acid is neutralized, and this results in the production of an effluent comprising mainly sulfates contaminated with acrylamide. This necessitates the highly poisonous acrylamide having to be crystallized from the residual water and handled as a powder.

The Applicant is also aware that the processes involving heterogeneous catalytic reaction, are prone to polymerization and separation problems when slurry-bed technology is used, while low concentrations of acrylamide in water, in the order of 7%, only are produced when single fixed bed reactors, i.e. not a series of reactors, are used. Phase separation limits the quantity of acrylonitrile that may be fed to the reactor with water. In this case, the catalyst, unreacted acrylonitrile and water have to be removed by filtration and/or distillation to reach a desired concentration of about 50%. Apart from being uneconomical as regards energy utilization (heat is removed in the reaction step and added again at the distillation stage), these processes are highly capital intensive as several reactors and distillation towers are required for purification and concentration of the product. Catalyst life time is also limited although the catalyst may in some instances be regenerated by oxidation followed by reduction with hydrogen.

The Applicant has surprisingly found that by applying catalytic distillation technology to the hydration of amides, many disadvantage of the known processes can be eliminated. The process of the invention is a continuous process, enabling large savings in capital cost (typically one reaction vessel against five reaction vessels with known processes) with little or no effluent production. A further advantage is that the heat of the reaction is partly used to heat the reactants, implying lower energy requirements. Since catalytic distillation is essentially a distillation process, controlling the reaction temperature and thus preventing or inhibiting unwanted polymerization, poses no difficulties. The required concentration of the product (50%) can also be reached without additional separation processes, and catalyst life time is enhanced. Little or no unwanted polymerization is experienced since the product is constantly removed from the heat source. Thus, an aqueous solution of the product at the desired concentration (1%-60%) can be obtained directly from the reactor with no extra purification or concentration being required, while energy requirements are minimized. In the case of olefinic nitriles, oligomerization/polymerization poses no problem if the pH is controlled between 3 and 8 as the product is constantly removed from the heat source.

Olefinic amides, e.g. acrylamides, methacrylamide, crotonamide, and 3-butenamide, which are prepared by the process of this invention can be used as monomers in polymerization reactions. For example, non-ionic and anionic polyacrylamides have been produced from acrylamides prepared by the process of the invention. It is believed that it will also be possible to produce, by means of the process of the invention, acrylamide suitable for the production of cationic polyacrylamides.

## Claims

1. A process for producing an amide and/or acid from a nitrile, which process comprises:
introducing a nitrile, as a first reactant, and a hydration compound, as a second reactant which is capable of reacting with the nitrile to convert it to its corresponding amide thus hydrating the nitrile and/or to convert it to its corresponding acid, into a treatment zone in a column or tower, with the catalyst bed provided in a section of the tower, and with a distillation zone being provided above and below the catalyst bed; and
comprising at least one reaction zone in which the hydration reaction of the nitrile to the amide and/or acid takes places catalytically in the presence of a catalyst, and at least one distillation zone adjacent the reaction zone in which distillation of the reaction product(s) from the reaction zone and/or unreacted reactants, takes place, with the reaction zone comprising a packed bed of particles of a copper or copper-based hydration catalyst;
effecting hydration of at least some of the nitrile to the corresponding amide, and/or its corresponding acid;
said hydration being effected simultaneously with distillation in a single treatment zone containing boiling liquid;
withdrawing the amide and/or acid from the treatment zone in a concentration of 1-60%.

2. A process according to claim 1, wherein the first reactant comprises an unsaturated or aromatic nitrile which is thus hydrated to the corresponding unsaturated or aromatic amide and/or acid.

3. A process according to claim 1 or 2, which includes boiling a liquid component in a reboiling zone operatively connected to a lower end of the treatment zone, to provide the driving boiling force for the catalytic distillation, with a portion of the liquid component optionally being introduced into the treatment zone above or below the catalyst bed.

4. A process according to claim 3, wherein the liquid component is such that it does not partake in the hydration reaction and only provides the driving force for the catalytic distillation, thus assisting in distillation of the reactants and products in the treatment zone, with the second reactant being fed into the treatment zone at a location spaced along the treatment zone from the point of introduction of the first reactant into the treatment zone.

5. A process according to claim 4, wherein the second reactant is water and wherein the liquid component is an organic compound.

6. A process according to claim 3, wherein the liquid component and the second reactant are water, so that the liquid component partakes in the hydration reaction.

7. A process according to claim 1 or 2, wherein the higher boiling of the first and second reactants is introduced into the treatment zone above the catalyst bed, with the lower boiling thereof being introduced below the catalyst bed.

8. A process according to claim 1 or 2, which includes boiling a liquid component in a reboiling zone operatively connected to a lower end of the treatment zone, to provide the driving force for the catalytic distillation, with a portion of the liquid component optionally being introduced into the treatment zone above or below the catalyst bed.

9. A process according to claim 1 or 2, wherein the higher boiling of the first and second reactants is introduced into the treatment zone above the catalyst bed, with the lower boiling thereof being introduced below the catalyst bed.

10. A process according to claim 3, wherein the higher boiling of the first and second reactants is introduced into the treatment zone above the catalyst bed, with the lower boiling thereof being introduced below the catalyst bed.

11. A process according to claim 4, wherein the higher boiling of the first and second reactants is introduced into the treatment zone above the catalyst bed, with the lower boiling thereof being introduced below the catalyst bed.

12. A process according to claim 5, wherein the higher boiling of the first and second reactants is introduced into the treatment zone above the catalyst bed, with the lower boiling thereof being introduced below the catalyst bed.

13. A process according to claim 6, wherein the higher boiling of the first and second reactants is introduced into the treatment zone above the catalyst bed, with the lower boiling thereof being introduced below the catalyst bed.

## Patentansprüche

1. Verfahren zum Herstellen eines Amids und/oder Säure aus einem Nitril, welches Verfahren umfasst:
Einführen eines Nitrils - als einen ersten reagierenden Stoff und eine Hydrat-Verbindung als einen zweiten reagierenden Stoff, der in der Lage ist, mit dem Nitril zu reagieren, um es in sein entsprechendes Amid umzuwandeln und damit das Amid zu hydratisieren und/oder es in die entsprechende Säure umzuwandeln - in eine Behandlungszone in einer Säule oder Kolonne, wobei das Katalysatorbett in einer Sektion in der Kolonne vorgesehen ist und wobei eine Destillationszone oberhalb und unterhalb des Katalysatorbettes vorgesehen ist; und
aufweisend mindestens eine Reaktionszone, in der die Reaktion der Hydratisierung des Nitrils zu dem Amid und/oder der Säure katalytisch in Gegenwart eines Katalysators stattfindet, und aufweisend mindestens eine Destillationszone angrenzend an der Reaktionszone, in der die Destillation des/der Reaktionsproduktes/Reaktionsprodukte aus der Reaktionszone und/oder die nichtumgesetzten reagierenden Stoffe stattfindet, wobei die Reaktionszone ein Füllkörperbett aus Partikeln eines Hydratisierungskatalysators aus Kupfer oder auf Kupfer-Basis aufweist;
Ausführen der Hydratation mindestens eines Teils des Nitrils zu dem entsprechenden Amid und/oder zu seiner entsprechenden Säure;
wobei die Hydratation gleichzeitig mit der Destillation in einer einzigen Behandlungszone ausgeführt wird, die siedende Flüssigkeit enthält;
Abziehen des Amids und/oder der Säure aus der Behandlungszone in einer Konzentration von 1 bis 60 %.

2. Verfahren nach Anspruch 1, bei welchem der erste reagierende Stoff ein ungesättigtes oder aromatisches Nitril aufweist, das so zu dem entsprechenden ungesättigten oder aromatischen Amid und/oder Säure hydratisiert wird.

3. Verfahren nach Anspruch 1 oder 2, welches Verfahren das Sieden einer flüssigen Komponente in einer Nachverdampfungszone einschließt, die funktionsfähig mit dem unteren Ende der Behandlungszone verbunden ist, um für die katalytische Destillation die treibende Kraft zum Sieden bereitzustellen, wobei ein Teil der flüssigen Komponente wahlweise in die Behandlungszone oberhalb oder unterhalb des Katalysatorbettes eingeführt wird.

4. Verfahren nach Anspruch 3, bei welchem die flüssige Komponente so beschaffen ist, dass sie nicht an der Reaktion der Hydratisierung teilnimmt und lediglich die treibende Kraft für die katalytische Destillation bereitstellt und so die Destillation der reagierenden Stoffe und Produkte in der Behandlungszone unterstützt, wobei der zweite reagierende Stoff in die Behandlungszone an einer Stelle eingeführt wird, die von der Einführungsstelle des ersten reagierenden Stoffes in die Behandlungszone beabstandet ist.

5. Verfahren nach Anspruch 4, bei welchem der zweite reagierende Stoff Wasser ist und worin die flüssige Komponente eine organische Verbindung ist.

6. Verfahren nach Anspruch 3, bei welchem die flüssige Komponente und der zweite reagierende Stoff Wasser sind, so dass die flüssige Komponente an der Reaktion der Hydratisierung teilnimmt.

7. Verfahren nach Anspruch 1 oder 2, bei welchem die höher Siedenden der ersten und zweiten reagierenden Stoffe in die Behandlungszone oberhalb des Katalysatorbettes eingeführt werden und deren niedriger Siedende davon unterhalb des Katalysatorbettes eingeführt werden.

8. Verfahren nach Anspruch 1 oder 2, welches Verfahren das Sieden einer flüssigen Komponente in einer Nachverdampfungszone einschließt, die funktionsfähig mit dem unteren Ende der Behandlungszone verbunden ist, um für die katalytische Destillation die treibende Kraft bereitzustellen, wobei ein Teil der flüssigen Komponente wahlweise in die Behandlungszone oberhalb oder unterhalb des Katalysatorbettes eingeführt wird.

9. Verfahren nach Anspruch 1 oder 2, bei welchem die höher Siedenden der ersten und zweiten reagierenden Stoffe in die Behandlungszone oberhalb des Katalysatorbettes eingeführt werden und deren niedriger Siedende davon unterhalb des Katalysatorbettes eingeführt werden.

10. Verfahren nach Anspruch 3, bei welchem die höher Siedenden der ersten und zweiten reagierenden Stoffe in die Behandlungszone oberhalb des Katalysatorbettes eingeführt werden und deren niedriger Siedende davon unterhalb des Katalysatorbettes eingeführt werden.

11. Verfahren nach Anspruch 4, bei welchem die höher Siedenden der ersten und zweiten reagierenden Stoffe in die Behandlungszone oberhalb des Katalysatorbettes eingeführt werden und deren niedriger Siedende davon unterhalb des Katalysatorbettes eingeführt werden.

12. Verfahren nach Anspruch 5, bei welchem die höher Siedenden der ersten und zweiten reagierenden Stoffe in die Behandlungszone oberhalb des Katalysatorbettes eingeführt werden und deren niedriger Siedende davon unterhalb des Katalysatorbettes eingeführt werden.

13. Verfahren nach Anspruch 6, bei welchem die höher Siedenden der ersten und zweiten reagierenden Stoffe in die Behandlungszone oberhalb des Katalysatorbettes eingeführt werden und deren niedriger Siedende davon unterhalb des Katalysatorbettes eingeführt werden.

## Revendications

1. Procédé de fabrication d'un amide et/ou d'un acide à partir d'un nitrile, lequel procédé consiste à :
- introduire un nitrile, en tant que premier réactif, et un composé d'hydratation, en tant que deuxième réactif, qui est capable de réagir avec le nitrile afin de le convertir en son amide correspondant, hydratant ainsi le nitrile, et/ou afin de le convertir en son acide correspondant, dans une zone de traitement dans une colonne ou une tour, le lit catalytique étant fourni dans une section de la tour, et une zone de distillation étant prévue au-dessus et au-dessous du lit catalytique ; et
- comprendre au moins une zone de réaction dans laquelle la réaction d'hydratation du nitrile en amide et/ou en acide a lieu, de façon catalytique, en présence d'un catalyseur, et au moins une zone de distillation adjacente à la zone de réaction, dans laquelle il se produit la distillation du ou des produits de réaction de la zone de réaction et/ou des réactifs qui n'ont pas réagis, la zone de réaction comprenant un lit à garnissage de particules d'un catalyseur d'hydratation de cuivre ou à base de cuivre ;
- effectuer l'hydratation d'au moins une partie du nitrile en amide correspondant, et/ou en acide correspondant ;
- effectuer ladite hydratation simultanément avec la distillation, dans une zone de traitement unique contenant le liquide en ébullition ;
- extraire l'acide et/ou l'amide de la zone de traitement, à une concentration de 1 à 60%.

2. Procédé selon la revendication 1, dans lequel le premier réactif comprend un nitrile insaturé ou aromatique, qui est ainsi hydraté en amide et/ou en acide correspondant(s), insaturé(s) ou aromatique(s).

3. Procédé selon la revendication 1 ou 2, qui comprend l'ébullition d'un composant liquide dans une zone de rebouillage, reliée fonctionnellement à une extrémité inférieure de la zone de traitement, afin de fournir la force motrice d'ébullition à la distillation catalytique, une partie du composant liquide étant facultativement introduite dans la zone de traitement au-dessus ou au-dessous du lit catalytique.

4. Procédé selon la revendication 3, dans lequel le composant liquide est tel, qu'il ne participe pas à la réaction d'hydratation et qu'il fournit seulement la force motrice à la distillation catalytique, aidant ainsi la distillation des réactifs et des produits dans la zone de traitement, le deuxième réactif étant alimente dans la zone de traitement à un emplacement situé à un intervalle, le long de la zone de traitement, du point d'introduction du premier réactif dans la zone de traitement.

5. Procédé selon la revendication 4, dans lequel le deuxième réactif est de l'eau, et dans lequel le composant liquide est un composé organique.

6. Procédé selon la revendication 3, dans lequel le composant liquide et le deuxième réactif sont de l'eau, de sorte que le composant liquide participe à la réaction d'hydratation.

7. Procédé selon la revendication 1 ou 2, dans lequel le réactif ayant un point d'ébullition supérieur, le premier ou le deuxième réactif, est introduit dans la zone de traitement au-dessus du lit catalytique, l'autre réactif, ayant un point d'ébullition inférieur, étant introduit au-dessous du lit catalytique.

8. Procédé selon la revendication 1 ou 2, qui comprend l'ébullition d'un composant liquide dans une zone de rebouillage, reliée fonctionnellement à une extrémité inférieure de la zone de traitement, afin de fournir la force motrice à la distillation catalytique, une partie du composant liquide étant facultativement introduite dans la zone de traitement au-dessus ou au-dessous du lit catalytique.

9. Procédé selon la revendication 1 ou 2, dans lequel le réactif ayant un point d'ébullition supérieur, le premier ou le deuxième réactif, est introduit dans la zone de traitement au-dessus du lit catalytique, l'autre réactif, ayant un point d'ébullition inférieur, étant introduit au-dessous du lit catalytique.

10. Procédé selon la revendication 3, dans lequel le réactif ayant un point d'ébullition supérieur, le premier ou le deuxième réactif, est introduit dans la zone de traitement au-dessus du lit catalytique, l'autre réactif, ayant un point d'ébullition inférieur, étant introduit au-dessous du lit catalytique.

11. Procédé selon la revendication 4, dans lequel le réactif ayant un point d'ébullition supérieur, le premier ou le deuxième réactif, est introduit dans la zone de traitement au-dessus du lit catalytique, l'autre réactif, ayant un point d'ébullition inférieur, étant introduit au-dessous du lit catalytique.

12. Procédé selon la revendication 5, dans lequel le réactif ayant un point d'ébullition supérieur, le premier ou le deuxième réactif, est introduit dans la zone de traitement au-dessus du lit catalytique, l'autre réactif, ayant un point d'ébullition inférieur, étant introduit au-dessous du lit catalytique.

13. Procédé selon la revendication 6, dans lequel le réactif ayant un point d'ébullition supérieur, le premier ou le deuxième réactif, est introduit dans la zone de traitement au-dessus du lit catalytique, l'autre réactif, ayant un point d'ébullition inférieur, étant introduit au-dessous du lit catalytique.
